# EUROPEAN PATENT APPLICATION

(11) **EP 3 108 798 A1**
(43) Date of publication of application: **28.12.2016**
(21) Application number: 15791981.2
(22) Date of filing: 11.05.2015
(51) Int. Cl.: A61B 1/00, A61B 1/04, A61B 1/06, G02B 23/24, G02B 23/26

(54) **ENDOSCOPIC SYSTEM**

(30) Priority: 16.05.2014 JP 2014102624
(71) Applicant: Olympus Corporation, Hachioji-shi Tokyo 192-8507 (JP)
(72) Inventor: HONDA Kazuki, Tokyo 192-8507 (JP)
(74) Representative: Gunzelmann, Rainer
(86) International application number: PCT/JP2015/063462
(87) International publication number: WO 2015/174365

(57) **Abstract**

An endoscope system includes an insertion portion 4, a front observation lens 12, a surrounding observation lens 13, an image generation section 60, an image processing section 64, a first illumination lens 15, second illumination lenses 24 and 25, and a filter driving section 65.

## Description

### Technical Field

The present invention relates to an endoscope system including: a first subject image acquisition portion configured to acquire a first subject image in a first direction; and a second subject image acquisition portion configured to acquire a second subject image in a second direction.

### Background Art

In recent years, endoscopes have been widely used in a medical field and an industrial field. The endoscope can observe inside of a subject by inserting an elongated insertion portion into the subject.

Note that examples of well-known endoscopes include: an already-known front-view type endoscope including an observation lens and an illumination lens provided on a distal end surface of a distal end portion provided on a distal end side of an insertion portion; and an already-known side-view type endoscope including an observation lens and an illumination lens provided on part of an outer circumferential surface of a distal end portion of an insertion portion.

Also well-known in recent years is an endoscope system including an endoscope for expanding an observation range in a subject, the endoscope being capable of observing not only a field of view in front of a distal end surface of an insertion portion in a first direction along a longitudinal direction of an insertion portion, but also at the same time a field of view in a circumference direction positioned around an outer circumferential surface of the distal end portion in a second direction that is substantially orthogonal to the first direction and that is a radial direction of the distal end portion. The endoscope system is disclosed in Japanese Patent No. 4782900 and U.S. Patent Application Publication No. 2013/0109916.

The endoscope system disclosed in Japanese Patent No. 4782900 includes a cylindrical portion protruding forward from the distal end surface of the distal end portion of the insertion portion of the endoscope, and a front observation lens that is a first subject image acquisition portion configured to observe forward is provided to face a distal end surface of the cylindrical portion in the cylindrical portion. In the cylindrical portion, a surrounding observation lens that is a second subject image acquisition portion is provided in a round shape along an outer circumferential surface of the cylindrical portion, behind the front observation lens.

In the distal end portion, a lens group is positioned behind the circumference direction observation lens, and an image pickup section, such as a CCD, is positioned at a light condensing position of the lens group.

A first subject positioned in front of the distal end surface is acquired as a first subject image by the front observation lens. Light entering the front observation lens passes through the surrounding observation lens, and the back lens group forms an image on the image pickup section.

A second subject positioned in the circumference direction of the cylindrical portion is acquired as a second subject image by the surrounding observation lens. The light entering the surrounding observation lens is reflected for a plurality of times in the lens, and the back lens group forms an image on the image pickup section.

Note that on the display section, the first subject image is displayed in a substantially circular shape, and the second subject image is displayed in a substantially annular shape so as to surround an outer circumference of the first subject image.

As a result, an operator can observe not only the field of view in front of the distal end portion of the insertion portion from the display section, but also the field of view in the circumference direction of the distal end portion at the same time. That is, a wide range in the subject can be observed.

Furthermore, first illumination lenses configured to respectively illuminate the first subject positioned in front are provided on the distal end surface of the distal end portion and on a support portion protruding forward from the distal end surface, and second illumination lenses configured to illuminate the second subject positioned in the circumference direction are provided on the outer circumferential surface of the cylindrical portion.

Illuminating light is supplied from a light source to each of the first illumination lenses and the second illumination lenses through a light guide inserted into the insertion portion.

In the endoscope system disclosed in U.S. Patent Application Publication No. 2013/0109916, a front observation lens that is a first subject image acquisition portion configured to observe forward is provided to face the distal end surface of the distal end portion of the insertion portion of the endoscope, and two surrounding observation lenses that are second subject image acquisition portions are provided on the outer circumferential surface of the distal end portion.

A first subject positioned in front of the distal end surface is acquired as a first subject image by the front observation lens, and an image is picked up by a first image pickup section, such as a CCD, provided in the distal end portion. A second subject positioned in the circumference direction of the distal end portion is acquired as second subject images by the respective surrounding observation lenses, and images are picked up by second image pickup sections, such as CCDs, each provided in the distal end portion.

Note that the second subject images are displayed on both sides of the first subject image on the display section. As a result, the operator can observe not only the field of view in front of the distal end portion of the insertion portion from the display section, but also the field of view in the circumference direction of the distal end portion at the same time.

Light-emitting elements for front observation, such as LEDs, configured to illuminate the first subject positioned in front are further provided on the distal end surface of the distal end portion, and light-emitting elements for surrounding observation, such as LEDs, configured to illuminate the second subject positioned in the circumference direction are also provided on the outer circumferential surface of the distal end portion.

By the way, to improve an observation property of the first subject and the second subject across a wide range in the subject, a maximum amount of illuminating light is generally supplied into the subject from the first illumination lenses and the second illumination lenses in Japanese Patent No. 4782900 and from the light-emitting elements for front observation and surrounding observation in U.S. Patent Application Publication No. 2013/0109916. Note that a configuration including a diaphragm blade configured to adjust the amount of light supplied from the light source to the light guide is disclosed in Japanese Patent No. 4782900.

Here, to sufficiently secure the field of view necessary for a portion of interest, a display ratio of the first subject image and the second subject image can be changed. In this case, if the maximum amount of illuminating light is supplied to the first subject and the second subject, the maximum amount of illuminating light is supplied to the field of view with a not so high display ratio. The illuminating light is wasted, and inside of the distal end portion may be quickly heated by energy of the illuminating light. Therefore, a scheme is necessary to prevent generation of a malfunction in a component provided on the distal end portion.

The same applies to the case of using the diaphragm blade described above. A light guide for first illumination lens supply and a light guide for second illumination lens supply are usually branched off from one light guide on a light source side. Therefore, for example, when only the field of view in front needs to be displayed, the maximum amount of illuminating light has to be supplied forward. There is a problem that an adjustment cannot be made by, for example, using the diaphragm blade to stop down the amount of supplied light.

The present invention has been made in view of the circumstances and the problem, and an object of the present invention is to provide an endoscope system capable of supplying appropriate amounts of illuminating light to a first subject positioned in front of a distal end portion of an insertion portion and a second subject positioned in a circumference direction of the distal end portion to thereby prevent overheating inside of the distal end portion.

### Disclosure of Invention

### Means for Solving the Problem

An aspect of the present invention provides an endoscope system including: an insertion portion inserted into a subject; a first subject image acquisition portion provided on the insertion portion and configured to acquire a first subject image from a first region of the subject; a second subject image acquisition portion provided on the insertion portion and configured to acquire a second subject image from a second region of the subject at least partially different from the first region; an image generation section configured to generate a first image from the first subject image and generate a second image from the second subject image; an image processing section configured to receive setting information related to sizes of the first image and the second image and execute a process of changing the sizes of the first image and the second image displayed on a display section based on the setting information; a first illumination portion configured to emit first illuminating light to the first region; a second illumination portion configured to emit second illuminating light to the second region; and an illumination control section configured to set a sum of amounts of the illuminating light respectively emitted to the first region and the second region to equal to or smaller than a predetermined amount and set a difference between the amounts of the first illuminating light and the second illuminating light to a relationship according to a magnitude correlation between the first image and the second image displayed on the display section.

### Brief Description of the Drawings

Fig. 1 is a perspective view showing a first embodiment and schematically showing an example of an endoscope apparatus including an endoscope and a peripheral apparatus;
Fig. 2 is an enlarged perspective view of a distal end portion of Fig. 1;
Fig. 3 is a plan view of the distal end portion of Fig. 2 as viewed in a direction III in Fig. 2;
Fig. 4 is a diagram schematically showing a configuration in the distal end portion, a cylindrical portion, and a support portion of Fig. 2 along with a light guide, a light amount adjustment filter, and a light source;
Fig. 5 is a diagram showing a state in which a first image and a second image are displayed on a monitor of Fig. 1;
Fig. 6 is a diagram schematically showing, along with the endoscope, a video processor, a light source apparatus, and the monitor, a configuration of changing sizes of the first image and the second image displayed on the monitor of Fig. 1 and a configuration of changing a difference between amounts of illuminating light respectively supplied from a first illumination lens and a second illumination lens to a relationship according to a magnitude correlation between the first image and the second image displayed on a display section;
Fig. 7 is a chart showing a plurality of patterns of a distribution ratio of the sizes of the first image and the second image provided in a storage section of Fig. 6;
Fig. 8 is a chart showing drawings of respective actions of a mode detection section, a ratio setting section, an image processing section, and a filter driving section of Fig. 6;
Fig. 9 is a diagram showing a modification in which a mode changeover switch of Fig. 1 is formed by a slider mechanism;
Fig. 10 is a diagram showing a modification in which the mode changeover switch of Fig. 1 is formed by a touch pad mechanism;
Fig. 11 is a diagram showing a modification of a filter of Fig. 4 along with a proximal end of the light guide;
Fig. 12 is a partial perspective view of a distal end portion of an insertion portion of an endoscope in an endoscope system according to a second embodiment;
Fig. 13 is a diagram showing a state in which a first image and second images are displayed on the monitor of the endoscope system of the second embodiment;
Fig. 14 is a diagram schematically showing, along with the endoscope, the video processor, the light source apparatus, and the monitor, a configuration of changing sizes of the first image and the second images displayed on the monitor of Fig. 1 and a configuration of changing a difference between amounts of illuminating light respectively supplied from the first illumination lens and the second illumination lens to a relationship according to a magnitude correlation between the first image and the second images displayed on the display section;
Fig. 15 is a chart showing drawings of respective actions of the ratio setting section, the image processing section, and a power adjustment section of Fig. 14;
Fig. 16 is a diagram showing a modification in which a keyboard of Fig. 14 is formed from a slider mechanism;
Fig. 17 is a partial perspective view of the distal end portion showing a modification in which the number of first light-emitting elements provided on a distal end surface of the distal end portion of the insertion portion of Fig. 12 and the number of second light-emitting elements provided on an outer circumference surface of the distal end portion of the insertion portion are increased from Fig. 12;
Fig. 18 is a perspective view schematically showing a modification in which an image acquisition unit can be attached to and detached from the insertion portion of an endoscope;
Fig. 19 is a diagram showing a modification of displaying the images by superimposing part of the second images on part of the first image displayed on the monitor of Fig. 13; and
Fig. 20 is a diagram showing a modification of displaying one image on each of a plurality of monitors.

### Best Mode for Carrying Out the Invention

Hereinafter, embodiments of the present invention will be described with reference to the drawings. Note that the drawings are schematic drawings, and a relationship between a thickness and a width of each member, a ratio of the thicknesses of respective members, and the like are different from reality. It is obvious that the relationship and the ratio of dimensions between the drawings are different in some parts of the drawings.

### (First Embodiment)

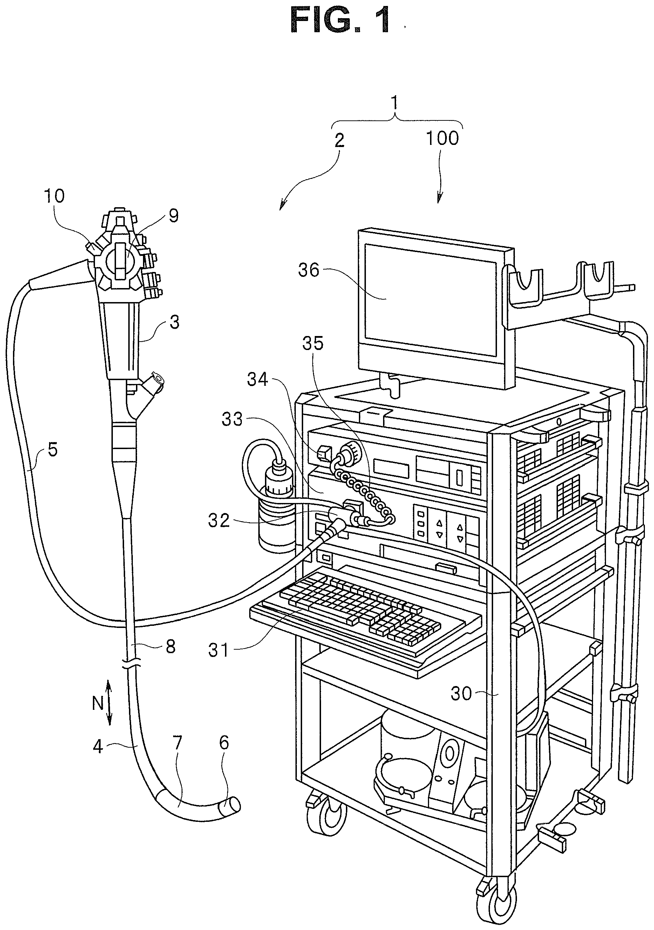

Fig. 1 is a perspective view showing the present embodiment and schematically showing an example of an endoscope apparatus including an endoscope and a peripheral apparatus.

As shown in Fig. 1, the endoscope system 1 includes an endoscope 2 and a peripheral apparatus 100.

Main components of the endoscope 2 include: an insertion portion 4 inserted into a subject; an operation portion 3 consecutively connected to a proximal end of the insertion portion 4 in a longitudinal direction N (hereinafter, simply called a proximal end); a universal cord 5 extended from the operation portion 3; and a connector 32 provided on an extension end of the universal cord 5.

The peripheral apparatus 100 includes: a keyboard 31; a light source apparatus 33; a video processor 34; a connection cable 35 electrically connecting the light source apparatus 33 and the video processor 34; and a monitor 36 that is a display section, which are mounted on a stand 30.

The endoscope 2 and the peripheral apparatus 100 with these components are connected to each other through the connector 32.

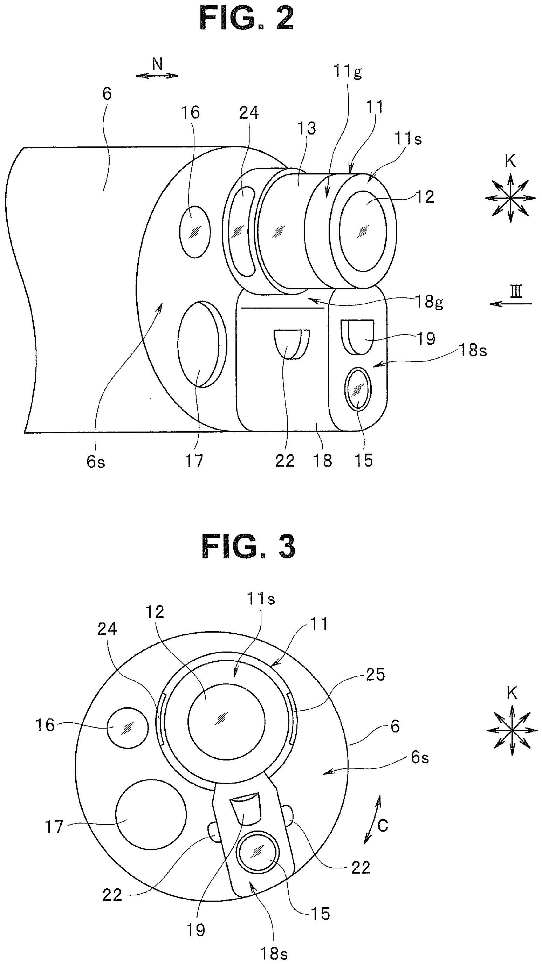

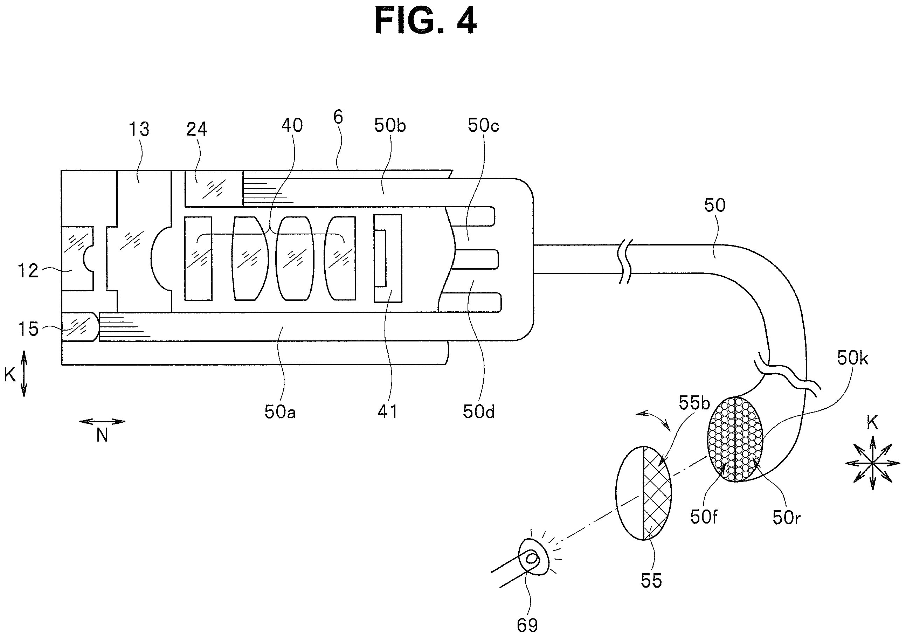

The connector 32 is connected to, for example, the light source apparatus 33 of the peripheral apparatus 100. The connector 32 is provided with: a pipe sleeve not shown connected with a proximal end of a treatment instrument insertion channel 17 (see Fig. 3) provided in the insertion portion 4, the operation portion 3, and the universal cord 5; a light guide pipe sleeve not shown forming a proximal end of a light guide 50 (see Fig. 4) described later inserted into the insertion portion 4, the operation portion 3, and the universal cord 5; an electric contact portion; and the like.

A bending operation knob 9 and a mode changeover switch 10 are provided on the operation portion 3 of the endoscope 2.

The insertion portion 4 of the endoscope 2 includes: a distal end portion 6 positioned on a distal end side of the insertion portion 4 in the longitudinal direction N (hereinafter, simply called a distal end side); a bending portion 7 consecutively connected to a proximal end of the distal end portion 6; and a flexible tube portion 8 consecutively connected to a proximal end of the bending portion 7.

The bending portion 7 is bent and operated in, for example, four vertical and horizontal directions by the bending operation knob 9 provided on the operation portion 3.

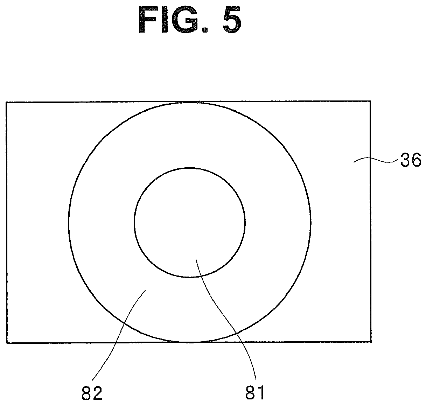

Next, a configuration of the distal end portion 6 will be described with reference to Figs. 2 to 5. Fig. 2 is an enlarged perspective view of the distal end portion of Fig. 1. Fig. 3 is a plan view of the distal end portion of Fig. 2 as viewed in a direction III in Fig. 2. Fig. 4 is a diagram schematically showing a configuration in the distal end portion, a cylindrical portion, and a support portion of Fig. 2 along with a light guide, a light amount adjustment filter, and a light source. Fig. 5 is a diagram showing a state in which a first image and a second image are displayed on the monitor of Fig. 1.

As shown in Fig. 2, a cylindrical portion 11 protruding forward in the longitudinal direction N (hereinafter, simply referred to as forward) from a position eccentric to a center of a distal end surface 6s in a radial direction K is provided on the distal end surface 6s of the distal end portion 6.

As shown in Figs. 2 to 4, a front observation lens 12 that is a first subject image acquisition portion configured to acquire a first subject image in a first direction substantially parallel to the longitudinal direction N, that is, from a first region of the subject, is provided in the cylindrical portion 11 so as to expose to a distal end surface 11s of the cylindrical portion 11.

Note that the first subject image is a subject image of a first subject positioned in the longitudinal direction N including in front of the distal end surface 11s. Therefore, the front observation lens 12 forms a front object image acquisition portion configured to acquire the first subject image in front in the longitudinal direction N.

As shown in Fig. 2, a surrounding observation lens 13 that is a second subject image acquisition portion exposed in a round shape along an outer circumferential surface 11g of the cylindrical portion 11 and configured to acquire a second subject image in a second direction at least partially different from the front in the longitudinal direction N (first region), that is, in a circumference direction that is a second region of the subject, is provided in the cylindrical portion 11.

Note that in the cylindrical portion 11, the surrounding observation lens 13 is positioned behind the front observation lens 12 in the longitudinal direction N (hereinafter, simply referred to as behind) as shown in Fig. 4.

The second subject image is a subject image of a second subject positioned in a circumference direction of the longitudinal direction N that is a radial direction K which is a direction intersecting the longitudinal direction N, such as substantially orthogonal to the longitudinal direction N. Therefore, the surrounding observation lens 13 forms a circumference direction object image acquisition portion configured to acquire the second subject image in the circumference direction. Note that reference sign K will also be provided to the circumference direction.

In the cylindrical portion 11, a back lens group 40 including a plurality of lenses is provided behind the surrounding observation lens 13 as shown in Fig. 4, and an image pickup section 41, such as a CCD, is provided at an image formation position of the back lens group 40.

The image pickup section 41 picks up the first subject image acquired by the front observation lens 12 and the second subject image acquired by the surrounding observation lens 13.

Note that a configuration for receiving light of the first subject entering the image pickup section 41 through the front observation lens 12, the surrounding observation lens 13, and the back lens group 40 and a configuration for receiving light of the second subject through the surrounding observation lens 13 and the back lens group 40 are well-known, and the details will not be described.

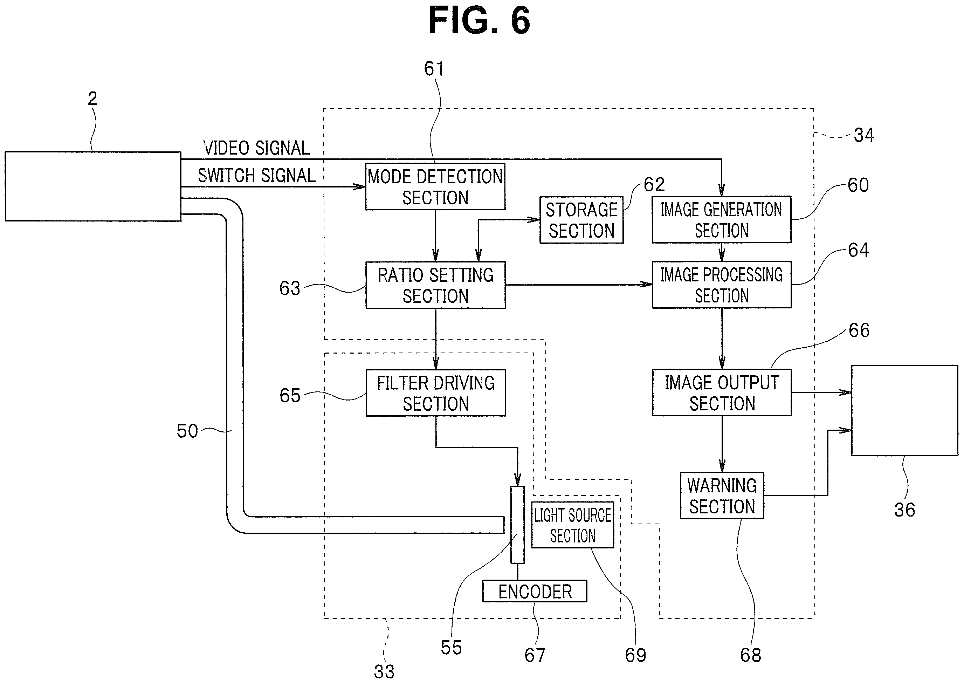

The image pickup section 41 is electrically connected to an image generation section 60 and an image processing section 64 (see Fig. 6) described later.

For the first subject image and the second subject image picked up by the image pickup section 41, the image generation section 60 generates a first image 81 from the first subject image and generates a second image 82 from the second subject image.

The image processing section 64 applies image processing to the first image 81 and the second image 82. A display image signal is generated by an image output section 66 (see Fig. 6) described later and is outputted to the monitor 36. As shown in Fig. 5, the first image 81 based on the first subject image is displayed in a substantially circular shape at a center of the monitor 36, and the second image 82 based on the second subject image is displayed in a substantially annular shape so as to surround an outer circumference of the first image 81.

As shown in Figs. 2 and 3, two second illumination lenses (second illumination portions) 24 and 25 that are illuminating light supply portions configured to supply illuminating light in the circumference direction K are provided on the outer circumferential surface 11 g of the cylindrical portion 11, behind the surrounding observation lens 13, and the second illumination lenses 24 and 25 are shifted by, for example, substantially 180° in a circumferential direction C as shown in Fig. 3. Note that the number of second illumination lenses is not limited to two. The number of second illumination lens may be three or more, or one illumination lens with a curved surface extending in the circumferential direction C may be provided, for example.

As shown in Fig. 4, the light guide 50 inserted into the insertion portion 4, the operation portion 3, the universal cord 5, and the connector 32 is partitioned into a first supply region 50f for supplying illuminating light in a first direction and a second supply region 50r for supplying illuminating light in a second direction, in the radial direction K.

The first supply region 50f is branched into light guides 50a and 50d at a midway position of the light guide 50 in the longitudinal direction, and the second supply region 50r is branched into light guides 50b and 50c at a midway position of the light guide 50 in the longitudinal direction.

Note that as shown in Fig. 4, distal ends of the light guides 50b and 50c in the longitudinal direction N (hereinafter, simply called distal ends) are positioned near proximal ends of the second illumination lenses 24 and 25 (the second illumination lens 25 is not illustrated in Fig. 4).

Therefore, illuminating light supplied from a light source section 69, which is configured to supply illuminating light applied to the subject, to the second supply region 50r through a proximal end 50k of the light guide 50 is supplied to the second illumination lenses 24 and 25 through the light guides 50, 50b, and 50c and is emitted in the circumference direction K through the second illumination lenses 24 and 25.

Note that an amount of light and an amount of heat of the illuminating light from the proximal end 50k to the second illumination lenses 24 and 25 through the light guides 50, 50b, and 50c vary depending on the number of fibers included in the light guides 50, 50b, and 50c.

As shown in Figs. 2 and 3, a support portion 18 adjacent to the cylindrical portion 11 and protruding forward is provided on the distal end surface 6s of the distal end portion 6.

A front-view observation window nozzle portion 19 configured to supply a fluid toward the front observation lens 12 is provided on a distal end surface 18s of the support portion 18.

A first illumination lens (first illumination portion) 15 that is an illumination light supply portion configured to supply illuminating light in front of the distal end surface 18s is also provided on the distal end surface 18s.

Surrounding observation window nozzles 22 configured to supply a fluid toward the circumference direction observation lens 13 are provided on an outer circumferential surface 18g of the support portion 18, and the number of surrounding observation window nozzles 22 is two, for example. Note that the number of surrounding observation window nozzles 22 is not limited to two.

A distal end of the treatment instrument insertion channel 17 opens to the distal end surface 6s of the distal end portion 6.

A first illumination lens (first illumination portion) 16 that is an illuminating light supply portion configured to supply illuminating light in front of the distal end surface 6s may also be provided on the distal end surface 6s.

Note that as shown in Fig. 4, distal ends of the light guides 50a and 50d are positioned near the proximal ends of the first illumination lenses 15 and 16 (the first illumination lens 16 is not shown in Fig. 4).

Therefore, the illuminating light supplied from the light source section 69 to the first supply region 50f through the proximal end 50k of the light guide 50 is supplied to the first illumination lenses 15 and 16 through the light guides 50, 50a, and 50d and is emitted forward through the first illumination lenses 15 and 16.

Note that an amount of light and an amount of heat of the illuminating light from the proximal end 50k to the first illumination lenses 15 and 16 through the light guides 50, 50a, and 50d vary depending on the number of fibers included in the light guides 50, 50a, and 50d.

As shown in Fig. 4, a light amount adjustment filter 55 configured to switch the amount of illuminating light entering the proximal end 50k of the light guide 50 from the light source section 69 is provided between the proximal end 50k of the light guide 50 and the light source section 69, on a same axis as the light source section 69 and the proximal end 50k.

Note that a detailed configuration of the light amount adjustment filter 55 will be described later. Although not shown, an already-known diaphragm blade is provided between the light amount adjustment filter 55 and the proximal end 50k of the light guide 50.

Next, a configuration of changing a distribution ratio of sizes of the first image 81 and the second image 82 displayed on the monitor 36 of Fig. 1 and a configuration of changing a distribution ratio of the illuminating light supplied from the first illumination lenses 15 and 16 and the second illumination lenses 24 and 25 will be illustrated with reference to Figs. 6 to 8.

Fig. 6 is a diagram schematically showing, along with the endoscope, the video processor, the light source apparatus, and the monitor, a configuration of changing the sizes of the first image and the second image displayed on the monitor of Fig. 1 and a configuration of changing a difference between the amounts of illuminating light respectively supplied from the first illumination lens and the second illumination lens to a relationship according to a magnitude correlation between the first image and the second image displayed on the display section.

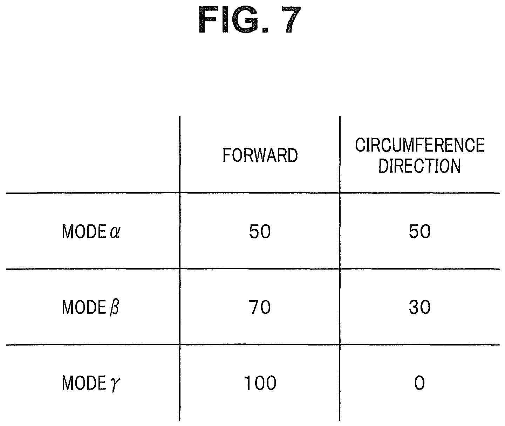

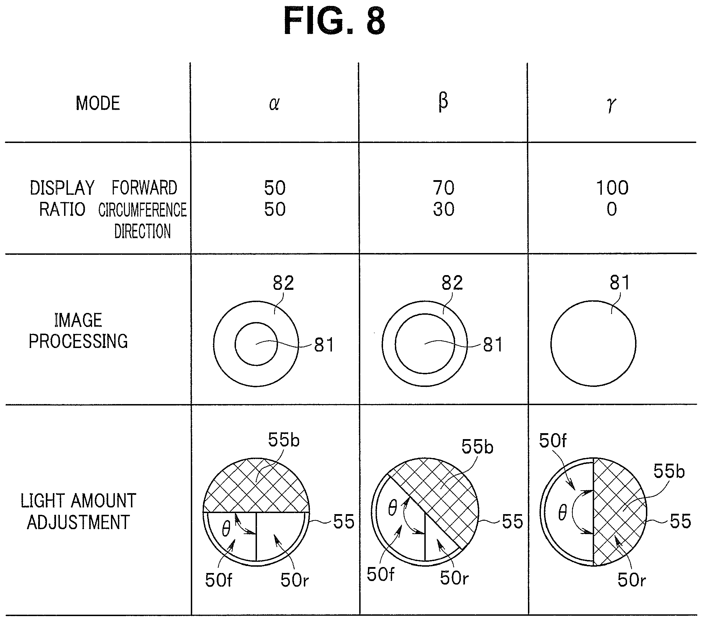

Fig. 7 is a chart showing a plurality of patterns of the distribution ratio of the sizes of the first image and the second image provided in a storage section of Fig. 6. Fig. 8 is a chart showing drawings of respective actions of a mode detection section, a ratio setting section, the image processing section, and a filter driving section of Fig. 6.

As shown in Fig. 6, a mode detection section 61, a storage section 62, the image generation section 60, an image distribution ratio setting section (hereinafter, simply called a ratio setting section) 63, the image processing section 64, the image output section 66, and a warning section 68 are provided in the video processor 34.

The light amount adjustment filter (hereinafter, simply called a filter) 55, a filter driving section 65 that is an illuminating light amount adjustment section (illumination control section), and an encoder 67 are provided in the light source apparatus 33.

As shown in Fig. 6, in response to a pressing operation of the mode changeover switch 10 shown in Fig. 1, the mode detection section 61 detects which one of a plurality of mode patterns, such as three mode patterns α, β, and γ, stored in the storage section 62 is selected as shown in Fig. 7.

Note that before the mode changeover switch 10 is pressed once, the mode pattern is switched from the mode pattern α to the mode pattern β, from the mode pattern β to the mode pattern γ, and from the mode pattern γ to the mode pattern α as shown in Figs. 7 and 8.

The mode detection section 61 detects the switch of the mode. Three mode changeover switches 10 may be separately provided on the operation portion 3 to allow individually selecting the three modes. The mode changeover switch 10 may also be provided on the video processor 34.

The mode patterns α, β, and γ stored in the storage section 62 indicate the distribution ratios of the sizes of the first image 81 and the second image 82 displayed on the monitor 36.

Note that as shown for example in Fig. 7, the distribution ratio of the first image (front image) : the second image (circumference direction image) is set to 50% : 50% in the mode pattern α in the present embodiment. The distribution ratio of the first image 81 : the second image 82 is set to 70% : 30% in the mode pattern β. The distribution ratio of the first image 82 : the second image 82 is set to 100% : 0% in the mode pattern γ.

The mode pattern α is used for, for example, observation in the subject. The mode pattern β is used for, for example, performing insertion into the subject by sufficiently securing the front field of view. The mode pattern γ is used for, for example, treatment of the first subject positioned in front of the insertion portion 4. Note that the field of view in the circumference direction is not necessary in the treatment of the first subject, and the distribution ratio of the second image 82 is 0%.

Note that the number of mode patterns stored in the storage section 62 is not limited to three. The distribution ratios of the mode patterns α, β, and γ are not limited to the examples, and the distribution ratio may be set according to preference of an operator.

A mode pattern in which the distribution ratio of the first image 81 : the second image 82 is 0% : 100% is excluded. This is because insertion work, observation work, and treatment performed by inserting the insertion portion 4 into the subject are not performed without the front field of view.

The ratio setting section 63 receives a detection result of the mode detection section 61 and sets the distribution ratio of the sizes of the first image 81 and the second image 82 displayed on the monitor 36.

More specifically, the ratio setting section 63 receives the detection result of the mode detection section 61 and sets the distribution ratio of the sizes of the first image 81 and the second image 82 displayed on the monitor 36 according to the pattern selected from the mode patterns α, β, and γ stored in the storage section 62 as shown in Fig. 8.

Note that the ratio setting section 63 receives the detection result of the mode detection section 61 and sets the distribution ratio of the size of the first image 81 displayed on the monitor 36 to greater than 0% and equal to or smaller than 100%.

The image generation section 60 generates a first image signal (first image) 81 from the first subject image picked up by the image pickup section 41 and generates a second image signal (second image) 82 from the second subject image. The image processing section 64 executes image processing of changing, that is, process of enlarging and reducing, the sizes of the first image 81 and the second image 82 displayed on the monitor 36 based on the distribution ratio set by the ratio setting section 63 as shown in Fig. 8.

More specifically, when the mode pattern α is selected, the image processing section 64 executes image processing of changing the sizes such that the first image 81 and the second image 82 are displayed on the monitor 36 at 50% : 50%.

When the mode pattern β is selected, the image processing section 64 executes image process of changing the sizes such that the first image 81 and the second image 82 are displayed on the monitor 36 at 70% : 30%.

When the mode pattern γ is selected, the image processing section 64 executes image processing of changing the sizes such that the first image 81 and the second image 82 are displayed on the monitor 36 at 100% : 0% (that is, only the first image 81 is displayed on the monitor 36).

Note that if the ratio setting section 63 sets the size of the first image 81 displayed on the monitor 36 to 0%, the image processing section 64 drives the warning section 68 to display a warning on the monitor 36.

Note that the warning may be a sound. In this case, the image processing section 64 does not execute the image processing of changing the size of the first image 81 displayed on the monitor 36.

The image output section 66 generates a display image signal to be displayed on the monitor 36 from one of (1) the first image signal subjected to the image processing by the image processing section 64 and (2) the first image signal and the second image signal and outputs the display image signal to the monitor 36.

Based on the magnitude correlation between the sizes of the first image 81 and the second image 82 displayed on the monitor 36, that is, the distribution ratio in the present embodiment, the filter driving section 65 sets the difference between the amounts of illuminating light supplied from the light source section 69 of the light source apparatus 33 and emitted forward and in the circumference direction K to the relationship according to the magnitude correlation between the first image 81 and the second image 82 displayed on the display section. In the present embodiment, the distribution ratio of the amounts of illuminating light supplied from the light source section 69 of the light source apparatus 33 and emitted forward and in the circumference direction K is changed.

More specifically, the filter driving section 65 includes the filter 55 shown in Fig. 4. The filter 55 selectively distributes the amounts of illuminating light to one of (1) the first supply region 50f of the light guide 50 and (2) the first supply region 50f and the second supply region 50r, or to one of the first supply region 50f and the second supply region 50r according to the distribution ratio of the amounts of illuminating light changed by the filter driving section 65.

More specifically, the filter 55 is formed in a substantially circular shape as shown in Fig. 4, and a mask 55b is provided in a half of the substantially circular shape. That is, the mask 55b has a substantially semicircular shape.

The filter 55 can be turned by the drive and the control of the filter driving section 65, and a turning angle θ of the filter 55 is detected by the encoder 67. Note that the turning angle θ is provided by θ=180×X/100 when the distribution ratio of the first image 81 is X%.

The mask 55b has a size that allows covering at least the entire second supply region 50r of the light guide 50 as shown in Fig. 8.

Therefore, as shown in Fig. 8, when the mode pattern α is detected, the filter driving section 65 turns the filter 55 to the turning position indicated in the mode pattern α of Fig. 8, in which the mask 55b of the filter 55 covers predetermined ranges of the respective regions 50f and 50r such that the illuminating light from the light source section 69 enters the first supply region 50f : the second supply region 50r with the distribution ratio of the amounts of light at 50% : 50%, through the proximal end 50k of the light guide 50.

When the mode pattern β is detected, the filter driving section 65 turns the filter 55 to the turning position indicated by the mode pattern β of Fig. 8, in which the mask 55b of the filter 55 covers predetermined ranges of the respective regions 50f and 50r such that the illuminating light from the light source section 69 enters the first supply region 50f : the second supply region 50r with the distribution ratio of the amounts of light at 70% : 30%, through the proximal end 50k of the light guide 50.

When the distribution ratio of the size of the first image 81 displayed on the monitor 36 is 100%, that is, when the mode pattern γ is detected, the filter driving section 65 turns the filter 55 to the turning position indicated by the mode pattern γ of Fig. 8, in which the mask 55b of the filter 55 covers only the region 50r such that the illuminating light from the light source section 69 enters the first supply region 50f : the second supply region 50r with the distribution ratio of the amounts of light at 100% : 0%, through the proximal end 50k of the light guide 50, that is, the illuminating light is supplied only forward.

As a result, the illuminating light with the distribution ratio of the amounts of light changed by the filter driving section 65 is supplied to one of (1) the first illumination lenses 15 and 16 and (2) the first illumination lenses 15 and 16 and the second illumination lenses 24 and 25 through the light guide 50 and is emitted into the subject.

In this way, in the present embodiment, the distribution ratio of the illuminating light emitted forward from the first illumination lenses 15 and 16 and the illuminating light emitted in the circumference direction K from the second illumination lenses 24 and 25 is changed according to the distribution ratio of the sizes of the first image 81 and the second image 82 displayed on the monitor 36.

The distribution ratio of the amounts of light of the illuminating light emitted forward from the first illumination lenses 15 and 16 and the illuminating light emitted in the circumference direction K from the second illumination lenses 24 and 25 is changed by turning the filter 55.

According to this, the distribution ratio of the illuminating light emitted forward and in the circumference direction K can be brought into line with the display ratio in the monitor 36 just by turning the filter 55 and changing the ranges of the first supply region 50f and the second supply region 50r of the light guide 50 covered by the mask 55b according to the distribution ratio of the sizes of the first image 81 and the second image 82 displayed on the monitor 36.

As a result, the illuminating light can be supplied forward and in the circumference direction K with the distribution ratio of the illuminating light according to the distribution ratio of the sizes of the first image 81 and the second image 82 for the monitor 36. Therefore, unlike in the conventional techniques, the illuminating light is not always supplied forward and in the circumference direction K at a maximum amount of light emission, and this can prevent the inside of the distal end portion 6 from being excessively heated.

Thus, appropriate amounts of illuminating light can be supplied to the first subject positioned in front of the distal end portion 6 of the insertion portion 4 and the second subject positioned in the circumference direction K of the distal end portion 6, and the endoscope system 1 capable of preventing overheating in the distal end portion 6 can be provided.

Note that a modification will be illustrated below.

In the present embodiment described above, the distribution ratio of the illuminating light emitted forward from the first illumination lenses 15 and 16 and the illuminating light emitted in the circumference direction K from the second illumination lenses 24 and 25 is changed according to the distribution ratio of the sizes of the first image 81 and the second image 82 displayed on the monitor 36.

However, the amount of illuminating light emitted forward and in the circumference direction K may be insufficient according to the configuration. For example, unlike in the conventional techniques, the illuminating light cannot be supplied forward and in the circumference direction K at 100% : 100%.

In this case, it is obvious that the image processing section 64 can individually adjust gains of brightness of the generated first image signal (first image 81) and second image signal (second image 82) to supplement the brightness.

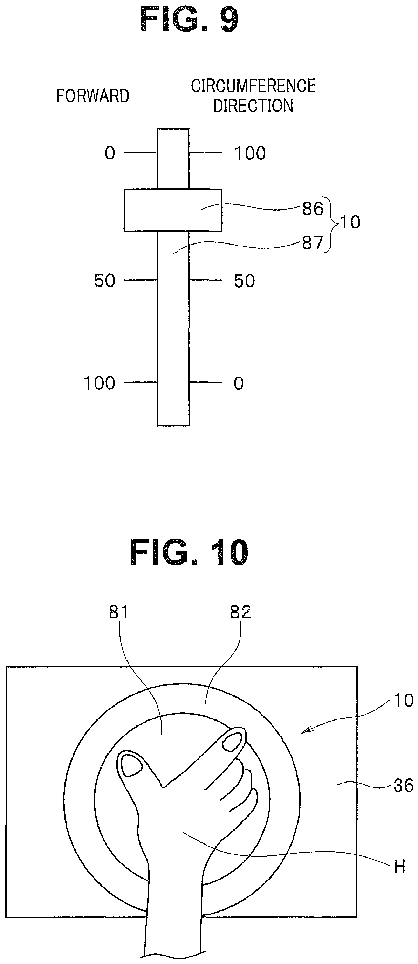

Another modification will be illustrated below with reference to Fig. 9. Fig. 9 is a diagram showing a modification in which the mode changeover switch of Fig. 1 is formed by a slider mechanism.

In the present embodiment described above, every time the mode changeover switch 10 provided on the operation portion 3 is pressed, the mode detection section 61 detects which mode pattern of the three modes stored in the storage section 62 is selected, and the ratio setting section 63 sets the distribution ratio of the sizes of the first image 81 and the second image 82 displayed on the monitor 36 according to the detected mode pattern.

The method is not limited to this, and the mode changeover switch 10 may be provided on a portion other than the operation portion 3 and may be formed by, for example, a slider mechanism including a slider 86 and a slider groove 87 provided on the video processor 34.

According to this, the operator can arbitrarily set the distribution ratio of the sizes of the first image 81 and the second image 82 displayed on the monitor 36 just by sliding and moving the slider 86 relative to the slider groove 87.

In this case, the mode detection section 61 can detect a position of the slider 86, and the ratio setting section 63 can set the distribution ratio of the sizes of the first image 81 and the second image 82 displayed on the monitor 36 according to a position detection result of the slider 86.

Note that when the distribution ratio of the sizes is arbitrarily set, the turning angle θ of the filter 55 is provided by θ=180×X/100 when the distribution ratio of the first image 81 is X% as described above. Therefore, the filter driving section 65 turns the filter 55 to the set turning angle θ based on the formula.

Another modification will be illustrated below with reference to Fig. 10. Fig. 10 is a diagram showing a modification in which the mode changeover switch of Fig. 1 is formed by a touch pad mechanism.

As shown in Fig. 10, the mode changeover switch 10 may also be a touch pad mechanism provided on the monitor 36.

According to this, the operator can arbitrarily set the distribution ratio of the sizes of the first image 81 and the second image 82 displayed on the monitor 36 just by using the touch pad mechanism to change the size of the first image 81 displayed on the monitor 36 by a finger H.

In this case, the mode detection section 61 can detect the size of the first image 81 on the monitor 36, and the ratio setting section 63 can set the distribution ratio of the sizes of the first image 81 and the second image 82 displayed on the monitor 36 according to a detection result of the size of the first image 81.

Note that the touch pad mechanism may be provided on a monitor different from the monitor 36. The mode changeover switch is not limited to the switch described above. The keyboard 31 may also serve as the mode changeover switch, or the mode changeover switch may be other input means using a mouse or the like.

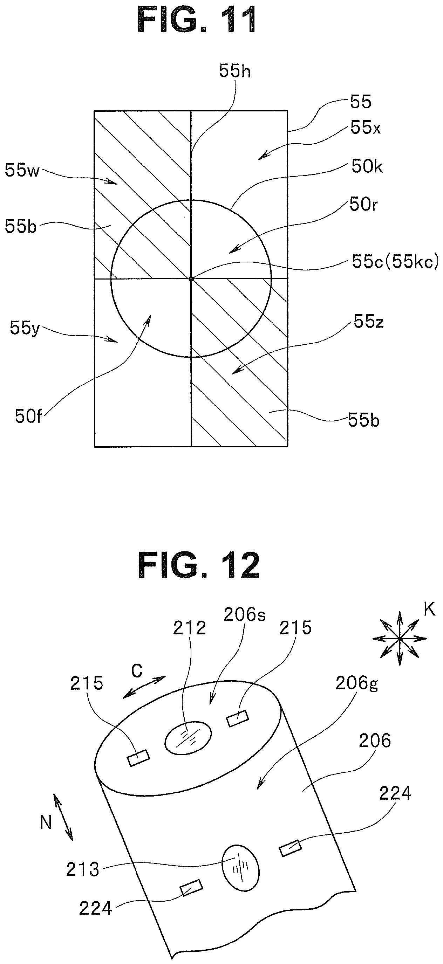

Another modification will be illustrated below with reference to Fig. 11. Fig. 11 is a diagram showing a modification of the filter of Fig. 4 along with a proximal end of the light guide.

In the present embodiment described above, the filter 55 has a substantially circular shape and includes the mask 55b in a substantially semicircular shape. The filter 55 can be turned.

The filter 55 is not limited to this, and as shown in Fig. 11, the filter 55 may be formed in a rectangular shape with a size covering the proximal end 50k of the light guide 50. The filter 55 may be equally divided into four regions 55w, 55x, 55y, and 55z, and the mask 55b may be formed only in the regions 55w and 55z positioned on a diagonal line. The filter 55 may be slid and moved in the vertical direction of Fig. 11. Note that the mask 55b may be formed only in the regions 55x and 55y positioned on a diagonal line.

According to the filter 55, in a state that the regions 55w and 55y are positioned on a left side in Fig. 11, and the regions 55x and 55z are positioned on a right side based on a border of the first supply region 50f and the second supply region 50r of the light guide 50 as shown in Fig. 11, the distribution ratio of the amount of illuminating light supplied from the light source section 69 to the first supply region 50f and the amount of illuminating light supplied from the light source section 69 to the second supply region 50r is 50% : 50% based on the mask 55w and the mask 55z, at a position where a center 55c of the filter 55 coincides with a center 55kc of the proximal end 50k.

Note that to increase the distribution ratio of the illuminating light supplied to the first supply region 50f, the filter 55 can be slid and moved upward in Fig. 11 from the position. To decrease the distribution ratio, the filter 55 can be slid and moved downward in Fig. 11 from the position.

According to the configuration of the filter 55, same effects as in the filter 55 shown in Fig. 4 can be obtained.

### (Second Embodiment)

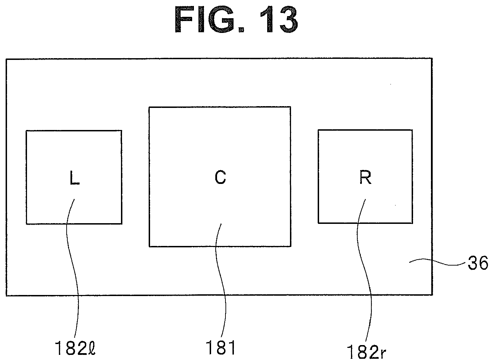

Fig. 12 is a partial perspective view of a distal end portion of an insertion portion of an endoscope in an endoscope system according to the present embodiment. Fig. 13 is a diagram showing a state in which a first image and second images are displayed on the monitor of the endoscope system of the present embodiment.

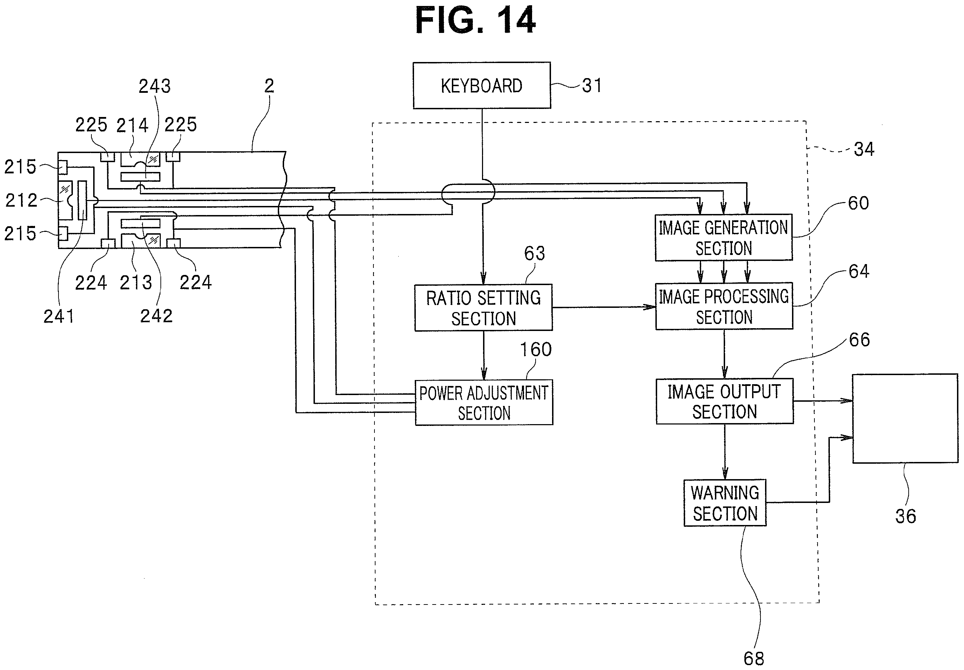

Fig. 14 is a diagram schematically showing, along with the endoscope, the video processor, the light source apparatus, and the monitor, a configuration of changing sizes of the first image and the second images displayed on the monitor of Fig. 1 and a configuration of changing a difference between amounts of illuminating light respectively supplied from the first illumination lens and the second illumination lens to a relationship according to a magnitude correlation between the first image and the second images displayed on the display section.

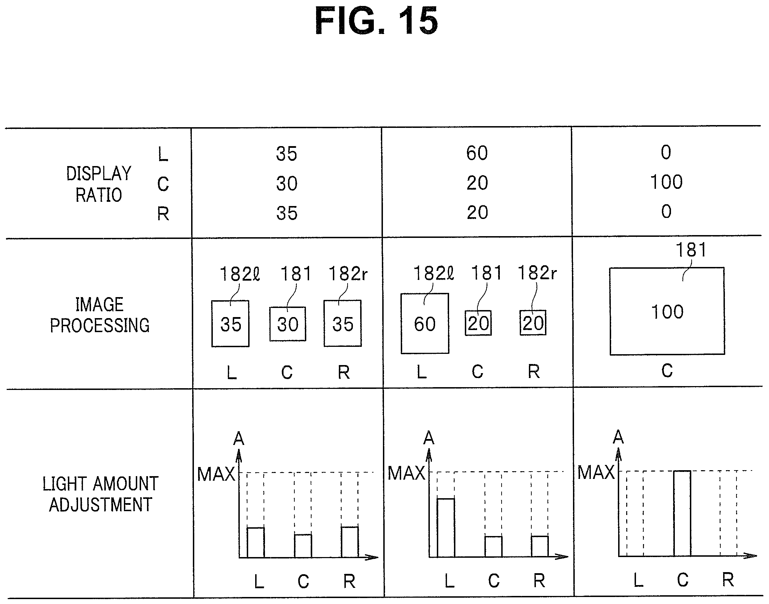

Fig. 15 is a chart showing drawings of respective actions of the ratio setting section, the image processing section, and a power adjustment section of Fig. 14.

The configuration of the endoscope system according to the second embodiment is different from the endoscope system according to the first embodiment shown in Figs. 1 to 8 in that image pickup sections are separately provided by a first observation lens and a second observation lens in the distal end portion, the light source section and the illuminating light supply portion are formed from light-emitting elements provided on the distal end portion, and the illuminating light amount adjustment section (illumination control section) is formed from a power adjustment section configured to adjust the amount of supply of power to the light-emitting elements.

Therefore, only the differences will be described. The same reference signs are provided to the same components as in the first embodiment, and the description will not be repeated.

As shown in Fig. 12, a front observation lens 212 that is a first subject image acquisition portion configured to acquire a first subject image from in front of a distal end surface 206s in the longitudinal direction N, that is, from the first region of the subject, is exposed to the distal end surface 206s of a distal end portion 206 of the insertion portion.

Note that the first subject image is a subject image of the first subject positioned in the longitudinal direction N including in front of the distal end surface 206s. Therefore, the front observation lens 212 forms a front object image acquisition portion configured to acquire the first subject image in front in the longitudinal direction N.

Two first light-emitting elements 215 that form light source sections configured to supply illuminating light emitted to the subject and that are illuminating light supply portions configured to supply illuminating light in front of the distal end surface 206s (first region of the subject) are provided on the distal end surface 206s. Note that the number of first light-emitting elements 215 is not limited to two.

Two surrounding observation lenses 213 and 214 that are second subject image acquisition portions configured to respectively acquire second subject images in a second direction at least partially different from the longitudinal direction N (first region), that is, the circumference direction K that is the second region of the subject, are provided on an outer circumferential surface 206g of the distal end portion 206 at equal angles, such as at an interval of 180°, in the circumferential direction C of the insertion portion.

Note that the number of surrounding observation lenses is not limited to two, and three or more surrounding observation lenses may be provided on the outer circumferential surface 206g at substantially equal angles in the circumferential direction C.

The second subject image is a subject image of the second subject positioned in the circumference direction that is the radial direction K which is a direction intersecting the longitudinal direction N, such as substantially orthogonal to the longitudinal direction N. Therefore, the surrounding observation lenses 213 and 214 form circumference direction object image acquisition portions configured to acquire the second subject image in the circumference direction.

Two second light-emitting elements 224 that form light source sections configured to supply illuminating light emitted to the subject and that are illuminating light supply portions configured to supply illuminating light in the circumference direction K (second region of the subject) are provided on the outer circumferential surface 206g, near the surrounding observation lens 213. Note that the number of second light-emitting elements 224 is not limited to two.

Two second light-emitting elements 225 that form light source sections configured to supply illuminating light emitted to the subject and that are illuminating light supply portions configured to supply illuminating light in the circumference direction K are further provided on the outer circumferential surface 206g, near the surrounding observation lens 214. Note that the number of second light-emitting elements 225 is not limited to two.

In the distal end portion 6, a first image pickup section 241, such as a CCD, configured to pick up an image of the first subject acquired by the front observation lens 212 is provided at an image formation position of the front observation lens 212.

In the distal end portion 6, a second image pickup section 242, such as a CCD, configured to pick up an image of the second subject acquired by the surrounding observation lens 213 is further provided at an image formation position of the surrounding observation lens 213. A second image pickup section 243, such as a CCD, configured to pick up an image of the second subject acquired by the surrounding observation lens 214 is provided at an image formation position of the surrounding observation lens 214.

The first image pickup section 241 and the second image pickup sections 242 and 243 are electrically connected to the image generation section 60 and the image processing section 64 (see Fig. 14).

As shown in Fig. 13, for the first subject picked up by the first image pickup section 241 and the second subject picked up by the second image pickup section, the image generation section 60 generates a first image 181 from the first subject image and generates second images 1821 and 182r from the second subject image.

The image processing section 64 applies image processing to the first image 181 and the second images 1821 and 182r. Display image signals are generated by the image output section 66 (see Fig. 14) described later and are outputted to the monitor 36. The first image 181 based on the first subject image is displayed at the center of the monitor 36, and the second images 1821 and 182r based on the second subject image are displayed adjacently to the first image 181, or more specifically, on both sides of the first image 181, separately from the first image 181.

Note that when three or more surrounding observation lenses are provided, the number of second image pickup sections provided corresponds to the number of surrounding observation lenses, and three or more second subject images are picked up, a plurality of second images are arranged and displayed on the monitor 36 at substantially equal angles in the circumferential direction C so as to surround the first image.

As shown in Fig. 14, the ratio setting section 63, the image processing section 64, a power adjustment section 160 that is an illuminating light amount adjustment section (illumination control section), the image output section 66, and the warning section 68 are provided in the video processor 34 in the present embodiment.

In the present embodiment, the ratio setting section 63 receives an input from the keyboard 31 or the like and sets a distribution ratio of sizes of the first image 181 and the second images 1821 and 182r displayed on the monitor 36.

Note that the ratio setting section 63 receives an input from the keyboard 31 or the like and sets the distribution ratio of the size of the first image 181 displayed on the monitor 36 to greater than 0% and equal to or smaller than 100%.

The ratio setting section 63 may set the distribution ratio of the sizes of the first image 181 and the second images 1821 and 182r displayed on the monitor 36 according to the pattern selected from the mode patterns α, β, and γ stored in the storage section 62 as in the first embodiment.

Note that in the following description of the present embodiment, three examples of the input from the keyboard 31 or the like will be described, the three examples including a case in which first image (C) : second image (L) : second image (R) is 30% : 35% : 35%, a case in which first image (C) : second image (L) : second image (R) is 20% : 60% : 20%, and a case in which first image (C) : second image (L) : second image (R) is 100% : 0% : 0% as shown in Fig. 15.

The operator may bring an ID card close to a read portion of the endoscope 2, the video processor 34, or the like. The distribution ratio of the sizes of the first image 181, the second image 1821, and the second image 182r preferred by the operator set for each operator stored in the storage section 62 may be read from the storage section 62 and set by the ratio setting section 63.

The image generation section 60 generates a first image signal (first image) 181 from the first subject image picked up by the first image pickup section 241 and generates a second image signal (second image) 82 from the second subject image picked up by the second image pickup sections 242 and 243. The image processing section 64 applies image processing of changing, that is, process of enlarging and reducing, the sizes of the first image 181 and the second images 1821 and 182r displayed on the monitor 36 based on the distribution ratio set by the ratio setting section 63 as shown in Fig. 15.

More specifically, when first image (C) : second image (L) : second image (R) is set to 30% : 35% : 35%, the image processing section 64 executes image processing of changing the sizes to display the first image 181 and the second images 1821 and 182r on the monitor 36 at 30% : 35% : 35%.

When first image (C) : second image (L) : second image (R) is set to 20% : 60% : 20%, the image processing section 64 executes image processing of changing the sizes to display the first image 181 and the second images 1821 and 182r on the monitor 36 at 20% : 60% : 20%.

When first image (C) : second image (L) : second image (R) is set to 100% : 0% : 0%, the image processing section 64 executes image processing of changing the sizes to display the first image 181 and the second images 1821 and 182r on the monitor 36 at 100% : 0% : 0% (that is, only the first image is displayed on the monitor 36).

Note that the image processing section 64 drives the warning section 68 to display a warning on the monitor 36 when the ratio setting section 63 sets the size of the first image 181 displayed on the monitor 36 to 0%.

Note that the warning may be a sound. In this case, the image processing section 64 does not execute the image processing of changing the size of the first image 181 displayed on the monitor 36.

The image output section 66 generates a display image signal to be displayed on the monitor 36 from one of (1) the first image signal subjected to the image processing by the image processing section 64 and (2) the first image signal and the second image signal and outputs the display image signal to the monitor 36.

Note that after a display percentage of each image is changed, the image output section 66 may immediately output each image to display the image with the changed distribution ratio on the monitor 36 or may output the image so as to gradually switch the image.

The distribution ratio may be displayed on the monitor 36 to allow the operator to immediately recognize the distribution ratio of the sizes of the respective subject images.

The power adjustment section 160 is electrically connected to the light-emitting elements 215, 224, and 225. Based on a magnitude correlation between the sizes of the first image 181 and the second images 1821 and 182r displayed on the monitor 36, that is, the distribution ratio in the present embodiment, the power adjustment section 160 sets a difference between the amounts of illuminating light supplied from the respective light-emitting elements 215, 224, and 225 and emitted forward and in the circumference direction K to a relationship according to a magnitude correlation between the first image 181 and the second images 1821 and 182r displayed on the display section. In the present embodiment, the distribution ratio of the amounts of illuminating light supplied from the respective light-emitting elements 215, 224, and 225 and emitted forward and in the circumference direction K is changed.

More specifically, the power adjustment section 160 adjusts amounts of operation energy for the respective light-emitting elements 215, 224, and 225 to emit the illuminating light.

More specifically, as shown in Fig. 15, the power adjustment section 160 adjusts the distribution ratio of amounts of current supply to the respective light-emitting elements 215, 224, and 225 for the respective light-emitting elements 215, 224, and 225 to emit the illuminating light according to the distribution ratio of the sizes of the first image 181 and the second images 1821 and 182r displayed on the monitor 36, based on maximum amounts of the light emission of the respective light-emitting elements 215, 224, and 225.

As a result, the power adjustment section 160 supplies the amounts of current with the adjusted distribution ratio to the respective light-emitting elements 215, 224, and 225, and the illuminating light with the changed distribution ratio of the amounts of light is emitted from one of (1) the first light-emitting element 215 and (2) the first light-emitting element 215 and the second light-emitting elements 224 and 225 into the subject.

Note that after a display percentage of each image is changed, the power adjustment section 160 may adjust the amounts of current to immediately change the distribution ratio of the amounts of illuminating light (amounts of light) emitted from the respective light-emitting elements 215, 224, and 225 or may adjust the amounts of current so as to gradually switch the distribution ratio.

The distribution ratio of the illuminating light may be displayed on the monitor 36 to allow the operator to immediately recognize the distribution ratio of the amounts of illuminating light.

The amounts of operation energy adjusted by the power adjustment section 160 are not limited to the amounts of current, but may be amounts of voltage or the like. That is, the amounts of power may be adjusted.

According to the configuration, the distribution ratio of the amount of illuminating light emitted forward from the first light-emitting elements 215 and the amounts of illuminating light emitted in the circumference direction K from the second light-emitting elements 224 and 225 is changed according to the distribution ratio of the sizes of the first image 181 and the second images 1821 and 182r displayed on the monitor 36. Therefore, the same effects as in the first embodiment can be obtained.

Note that a modification will be illustrated below.

In the present embodiment described above, the distribution ratio of the amount of illuminating light emitted forward from the first light-emitting elements 215 and the amount of illuminating light emitted in the circumference direction K from the second light-emitting elements 224 and 225 is changed according to the distribution ratio of the sizes of the first image 181 and the second images 182 displayed on the monitor 36.

However, the amount of illuminating light emitted forward and in the circumference direction K may be insufficient according to the configuration. For example, unlike in the conventional techniques, the illuminating light cannot be supplied forward and in the circumference direction K at 100% : 100%.

In this case, it is obvious that the image processing section 64 can individually adjust gains of brightness of the generated first image signal 181 and second image signals 1821 and 182r to supplement the brightness as in the first embodiment.

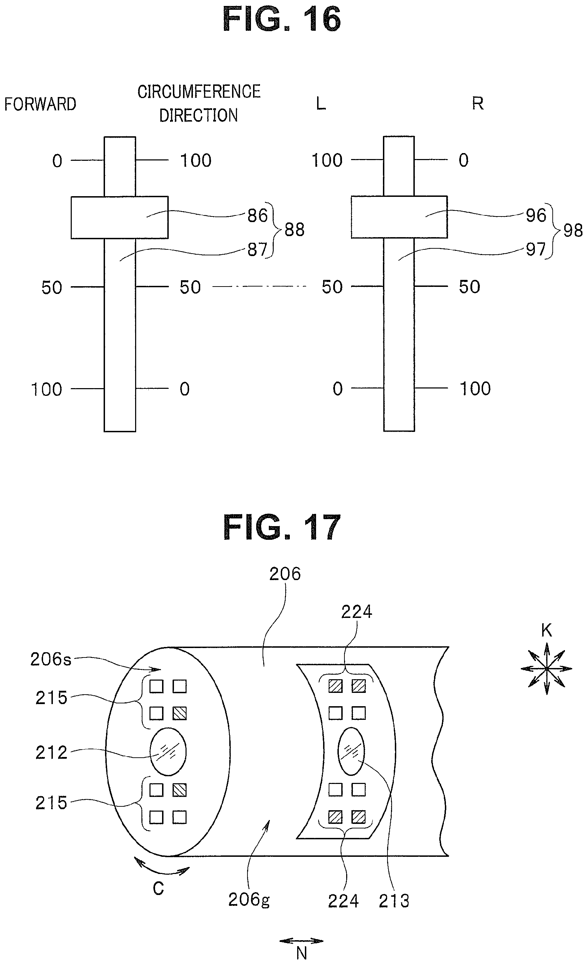

Another modification will be illustrated below with reference to Fig. 16. Fig. 16 is a diagram showing a modification in which the keyboard of Fig. 14 is formed from a slider mechanism.

In the present embodiment described above, the ratio setting section 63 receives an input from the keyboard 31 and sets the distribution ratio of the sizes of the first image 181 and the second images 1821 and 182r displayed on the monitor 36.

The distribution ratio is not limited to this, and the distribution ratio of the sizes of the first image 181 and the second images 1821 and 182r set by the ratio setting section 63 and displayed on the monitor 36 may be formed from, for example, a slider mechanism 88 including a slider 86 and a slider groove 87 provided on the video processor 34 and a slider mechanism 98 including a slider 96 and a slider groove 97 as shown in Fig. 16.

According to this, the operator can arbitrarily set the distribution ratio of the sizes of the first image 181 and the second images 1821 and 182r displayed on the monitor 36 just by sliding and moving the slider 86 relative to the slider groove 87. The operator can also arbitrarily set the distribution ratio of the sizes of the second image 1821 and the second image 182r displayed on the monitor 36 just by sliding and moving the slider 96 relative to the slider groove 97.

In this case, the ratio setting section 63 can set the distribution ratio of the sizes of the first image 181 and the second images 1821 and 182r displayed on the monitor 36 according to position detection results of the sliders 86 and 96.

Another modification will be illustrated below with reference to Fig. 17. Fig. 17 is a partial perspective view of the distal end portion showing a modification in which the number of first light-emitting elements provided on the distal end surface of the distal end portion of the insertion portion of Fig. 12 and the number of second light-emitting elements provided on the outer circumference surface of the distal end portion of the insertion portion are increased from Fig. 12.

In the present embodiment described above, the amounts of current supplied from the power adjustment section 160 to the respective light-emitting elements 215, 224, and 225 are adjusted to change the distribution ratio of the amount of illuminating light emitted forward from the first light-emitting elements 215 and the amount of illuminating light emitted in the circumference direction K from the second light-emitting elements 224 and 225 according to the distribution ratio of the sizes of the first image 181 and the second images 1821 and 182r displayed on the monitor 36.

The method is not limited to this, and as shown in Fig. 17, the numbers of first light-emitting elements 215 and second light-emitting elements 224 and 225 may be increased from Fig. 12, and the numbers of light-emitting elements that emit light may be different in the respective light-emitting elements 215, 224, and 225. In this way, the distribution ratio of the amount of illuminating light emitted forward from the first light-emitting elements 215 and the amounts of illuminating light emitted in the circumference direction K from the second light-emitting elements 224 and 225 may be changed.

The distribution ratio of the amount of illuminating light emitted forward from the first light-emitting element 215 and the amounts of illuminating light emitted in the circumference direction K from the second light-emitting elements 224 and 225 may be changed by adjusting amounts of emitted pulses of the respective light-emitting elements 215, 224, and 225.

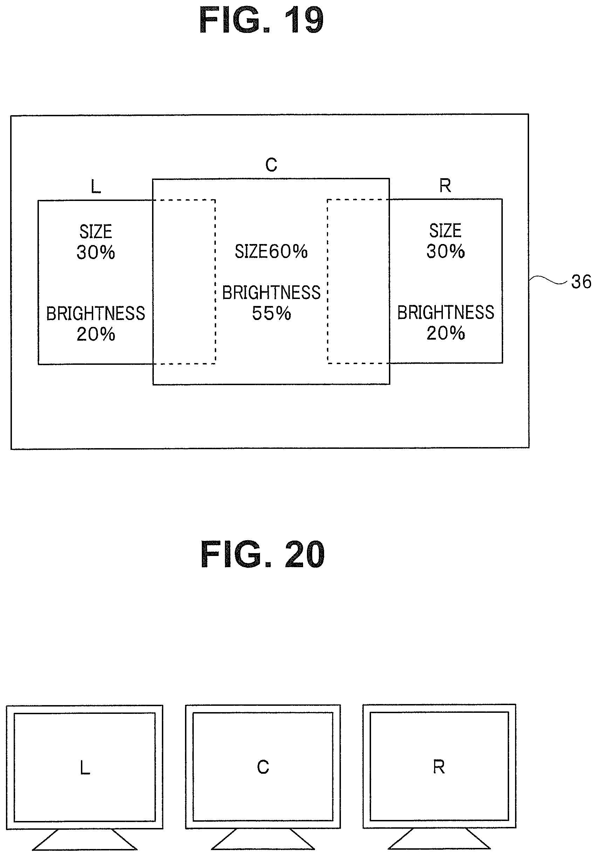

Fig. 19 is a diagram showing a modification of displaying the images by superimposing part of the second images on part of the first image displayed on the monitor of Fig. 13. Fig. 20 is a diagram showing a modification of displaying one image on each of a plurality of monitors.

Although one monitor displays a plurality of images in the examples illustrated in the first and second embodiments, the method is not limited to this, and it is obvious that one image may be displayed on each of a plurality of monitors as shown in Fig. 20, particularly in the second embodiment.

In the setting of the distribution ratio of the sizes of the first image 181 and the second images 1821 and 182r by the ratio setting section 63, a sum of the sizes of the first image and the second images 1821 and 182r is, for example, 100% in the distribution ratio, particularly in the example of the second embodiment.

The distribution ratio is not limited to this. The second images 1821 and 182r are displayed on the monitor 36, separately from the first image 181. Therefore, the distribution ratio is not limited to this, and the sum of the first image 181 and the second images 1821 and 182r does not have to be 100%.

That is, as described in Fig. 19, when an image of interest, such as the first image, is displayed in a size of 60%, each of two second images may be displayed in a size of 30%. Even when the sum of the sizes of the first image and the second images is not within, for example, 100%, the first image that is the image of interest may be superimposed in layers (stratification) over the two second images that are not images of interest. In this way, the image of interest may be displayed on a front side of the monitor, for example.

When the images are superimposed and displayed in layers (stratification) as described above, the images may be displayed on the monitor 36 such that the distribution ratio of the sizes of the first image 181, the second image 1821, and the second image 182r is 100% : 100% : 100%.

That is, as long as the total is within 300%, the operator can individually and freely set and input the display percentage of each image from the keyboard 31 or the like.

When the sum of the amounts of illuminating light respectively emitted to the first region (longitudinal direction N) and the second region (radial direction K) is set within a predetermined amount (for example, 100%), the illumination control section can set the difference between the amount of first illuminating light and the amount of second illuminating light to the relationship according to the magnitude correlation between the first image and the second images displayed on the display section.

For example, as described in Fig. 19, the illumination control section may perform setting of distributing a greatest amount of light (for example, 55%) to the region of interest, such as the first region, and distributing smaller amounts of light (for example, 20% each) to regions that are not the region of interest, such as the second regions, even when the sum of the distributions of the amounts of illuminating light is below 100%.

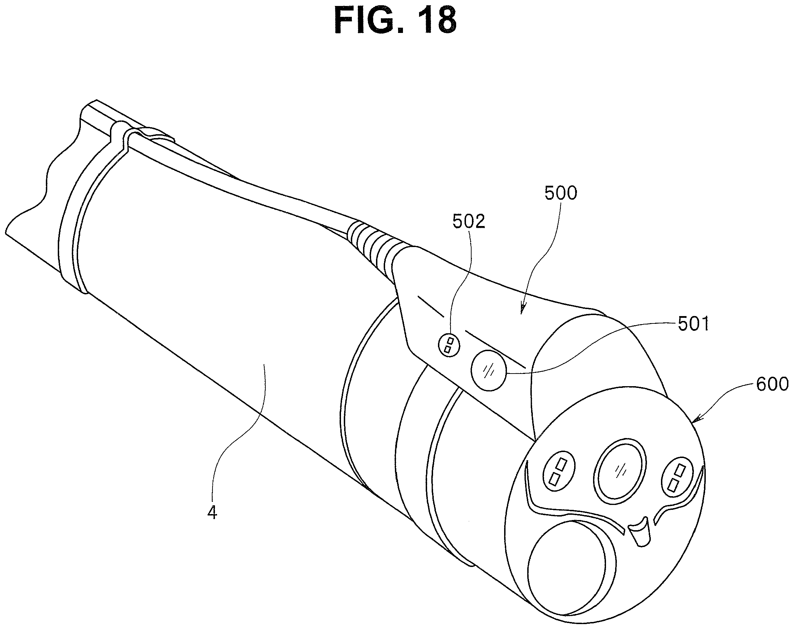

Note that a modification will be illustrated below with reference to Fig. 18. Fig. 18 is a perspective view schematically showing a modification in which an image acquisition unit can be attached to and detached from the insertion portion of an endoscope.

Note that as shown in Fig. 18, the embodiments can also be applied to a normal endoscope 600 configured to acquire a front first subject image, the endoscope 600 including a detachable image acquisition unit 500 including: second subject image acquisition portions 501 configured to acquire respective second subject images on left and right sides; and second illuminating light supply portions 502 configured to respectively illuminate the left and right sides.

The present application is filed on the basis of claiming the benefit of priority from Japanese Patent Application No. 2014-102624, filed on May 16, 2014 in Japan, and the contents are incorporated in the present specification, the claims, and the drawings by reference.

## Claims

1. An endoscope system comprising:
an insertion portion inserted into a subject;
a first subject image acquisition portion provided on the insertion portion and configured to acquire a first subject image from a first region of the subject;
a second subject image acquisition portion provided on the insertion portion and configured to acquire a second subject image from a second region of the subject at least partially different from the first region;
an image generation section configured to generate a first image from the first subject image and generate a second image from the second subject image;
an image processing section configured to receive setting information related to sizes of the first image and the second image and execute a process of changing the sizes of the first image and the second image displayed on a display section based on the setting information;
a first illumination portion configured to emit first illuminating light to the first region;
a second illumination portion configured to emit second illuminating light to the second region; and
an illumination control section configured to set a sum of amounts of the illuminating light respectively emitted to the first region and the second region to equal to or smaller than a predetermined amount and set a difference between the amounts of the first illuminating light and the second illuminating light to a relationship according to a magnitude correlation between the first image and the second image displayed on the display section.

2. The endoscope system according to claim 1, further comprising:
an image distribution ratio setting section configured to set a distribution ratio of the sizes of the first image and the second image displayed on the display section, wherein
the image processing section executes image processing of changing the sizes of the first image and the second image displayed on the display section based on the distribution ratio set by the image distribution ratio setting section.

3. The endoscope system according to claim 2, wherein
the image processing section sets the distribution ratio of the size of the first subject image displayed on the display section to greater than 0% and equal to or smaller than 100%, and
the illumination control section changes the distribution ratio of the amounts of light to emit the first illuminating light from the first illumination portion only for the first region when the distribution ratio of the size of the first subject image displayed on the display section is 100%.

4. The endoscope system according to claim 1, wherein
the illuminating light is supplied from a light source section configured to supply illuminating light emitted to the subject to one of the first illumination portion and the second illumination portion through a light guide, and
the illumination control section includes a light amount adjustment filter configured to switch an amount of the illuminating light entering the light guide from the light source section.

5. The endoscope system according to claim 4, wherein
the light guide is partitioned into a first supply region for supplying the illuminating light to the first region and a second supply region for supplying the illuminating light to the second region in a radial direction of the light guide, and
the light amount adjustment filter blocks predetermined ranges in the first supply region and the second supply region according to the distribution ratio of the illuminating light changed by the illumination control section to selectively distribute the amounts of the illuminating light to one of the first supply region, and the first supply region and the second supply region, or to one of the first supply region and the second supply region.

6. The endoscope system according to claim 1, wherein
the first illumination portion and the second illumination portion are formed by light-emitting elements configured to emit the illuminating light and are provided on the insertion portion, and
the illumination control section is formed by a power adjustment section configured to adjust an amount of operation energy for the light-emitting elements to emit the illuminating light.

7. The endoscope system according to claim 1, wherein
the display section configured to display the first image and the second image displays the second image adjacent to the first image.

8. The endoscope system according to claim 2, further comprising:
a storage section in which a plurality of patterns of the distribution ratio of the sizes of the first image and the second image are stored,
wherein the image distribution ratio setting section sets the distribution ratio of the sizes of the first image and the second image displayed on the display section according to one of the patterns selected from the storage section by an instruction from outside.

9. The endoscope system according to claim 1, wherein
the image processing section displays a warning on the display section and executes a process of not changing the size of the first image when the image distribution ratio setting section sets the size of the first image displayed on the display section to 0%.

10. The endoscope system according to claim 1, wherein
the first subject image is a subject image positioned in the first region substantially parallel to a longitudinal direction of the insertion portion, the first region including a front part of a distal end surface of the insertion portion,
the second subject image is a subject image positioned in the second region including a circumference direction that is a radial direction of the insertion portion which is a direction intersecting the longitudinal direction of the insertion portion,
the first subject image acquisition portion is a front object image acquisition portion configured to acquire the first subject image of the front part in the first region, and
the second subject image acquisition portion is a circumference direction object image acquisition portion configured to acquire the second subject image in the circumference direction in the second region.

11. The endoscope system according to claim 10, wherein
the first subject image acquisition portion is provided on the distal end surface of the insertion portion, the second subject image acquisition portion is provided in a circumferential direction of the insertion portion on an outer circumferential surface of the insertion portion, and
the insertion portion includes an image pickup section configured to pick up the first subject image acquired by the first subject image acquisition portion and the second subject image acquired by the second subject image acquisition portion, the image pickup section electrically connected to the image processing section.

12. The endoscope system according to claim 11, wherein
the display section configured to display the first image and the second image causes the first subject image to be displayed in a substantially circular shape and causes the second subject image to be displayed in a substantially annular shape so as to surround the first subject image.

13. The endoscope system according to claim 10, wherein
the first subject image acquisition portion is provided on the distal end surface of the insertion portion, and the second subject image acquisition portion is provided in a circumferential direction of the insertion portion on an outer circumferential surface of the insertion portion,
the endoscope system separately includes a first image pickup section configured to pick up the first subject image acquired by the first subject image acquisition portion and a second image pickup section configured to pick up the second subject image acquired by the second subject image acquisition portion, and
the first image pickup section and the second image pickup section are electrically connected to the image processing section.

14. The endoscope system according to claim 13, wherein
the second subject image acquisition portion is arranged in plurality at substantially equal angles in the circumferential direction of the insertion portion on the outer circumferential surface of the insertion portion, and
the image processing section causes the display section to display and arrange the first subject image at a center in the display section and to display and arrange the second subject image in plurality at substantially equal angles in the circumferential direction so as to surround the first subject image.

15. The endoscope system according to claim 1, wherein
the image processing section further individually adjusts gains of brightness of the first image and the second image.
